(19) Europäisches Patentamt

European Patent Office

Office européen des brevets

(11) Veröffentlichungsnummer: **0 307 673 B1**

# (12) EUROPÄISCHE PATENTSCHRIFT

(45) Veröffentlichungstag der Patentschrift: **13.05.92**

(21) Anmeldenummer: **88113816.8**

(22) Anmeldetag: **25.08.88**

(51) Int. Cl.⁵: **C07C 21/18**, C07C 17/38

(54) **Verfahren zur Gewinnung von reinem Tetrafluorethylen.**

(30) Priorität: **01.09.87 DE 3729106**

(43) Veröffentlichungstag der Anmeldung:
**22.03.89 Patentblatt 89/12**

(45) Bekanntmachung des Hinweises auf die
Patenterteilung:
**13.05.92 Patentblatt 92/20**

(84) Benannte Vertragsstaaten:
**DE GB IT NL**

(56) Entgegenhaltungen:
**DE-B- 1 161 877**
**DE-B- 1 295 543**

(73) Patentinhaber: **HOECHST AKTIENGESELL-SCHAFT**
**Postfach 80 03 20**
**W-6230 Frankfurt am Main 80(DE)**

(72) Erfinder: **Voigt, Hartmut**
**Burgfriedstrasse 10**
**W-8261 Winhöring(DE)**
Erfinder: **Freudenreich, Reinhold**
**Ortlehnerstrasse 1**
**W-8269 Burgkirchen(DE)**

**Beschreibung**

Die Erfindung betrifft ein Verfahren zur Gewinnung von reinem Tetrafluorethylen aus einem Gas, das bei der Pyrolyse einer Fluorkohlenstoffverbindung mit 1 bis 4 C-Atomen, die ein Wasserstoff- und ein Chloratom enthalten kann, entsteht, nach Anspruch 1.

Tetrafluorethylen wird im technischen Maßstab durch Pyrolyse von Fluorkohlenstoffverbindungen im allgemeinen im Bereich von 400 bis 1 000 °C gewonnen. Hierzu werden häufig Fluorkohlenstoffverbindungen mit 1 bis 4 C-Atomen eingesetzt, die neben C- und F-Atomen noch ein H-Atom sowie auch ein Cl- oder Br-Atom enthalten. Das bei der Pyrolyse entstehende Gasgemisch wird in der Regel von abgespaltenem HF; HCl oder HBr durch Waschen mit Wasser oder einer wäßrigen Lösung der genannten Stoffe entfernt, der Gasstrom getrocknet und kondensiert. Hierbei werden Tetrafluorethylen (nachfolgend mit TFE bezeichnet), nicht umgesetztes Ausgangsprodukt und unerwünschte Nebenprodukte verflüssigt, während Stickstoff, Kohlenoxid und entsprechend ihrem Partialdruck auch etwas TFE, andere Pyrolyseprodukte sowie auch Pyrolyse-Ausgangsprodukt gasförmig verbleiben. Das verflüssigte Produkt wird nun destillativ aufgearbeitet, wobei in einer ersten Kolonne die Verbindungen und azeotropen Gemische über Kopf abdestilliert werden, deren Siedepunkt unter den gewählten Bedingungen tiefer als der Siedepunkt des TFE liegt, die also leichter als TFE sieden (nachfolgend auch "Leichtsieder" genannt). Das Sumpfprodukt dieser ersten Kolonne wird dann in der Regel in einer zweiten Kolonne destilliert, aus der über Kopf mehr oder weniger reines TFE abdestilliert wird, während im Sumpf dieser Kolonne die Verbindungen und azeotropen Gemische verbleiben, deren Siedepunkt unter den gewählten Bedingungen höher als der Siedepunkt des TFE liegt, die also schwerer als TFE sieden (nachfolgend auch "Schwersieder" genannt). Die Schwersieder, gegebenenfalls auch die Leichtsieder, werden anschließend destillativ weiter aufgearbeitet, insbesondere, um das nicht umgesetzte Pyrolyse-Ausgangsprodukt möglichst rein wiederzugewinnen und in die Pyrolyse zurückzuführen.

Das Siedeverhalten verschiedener, neben TFE in dem flüssigen Pyrolysegas-Kondensat enthaltenen Verbindungen und azeotropen Gemische, ist für die Reindarstellung des TFE ungünstig und macht diese apparativ und bezüglich der erforderlichen Energie aufwendig. Um die destillative Aufarbeitung der Pyrolyseprodukte zu verbessern, sind verschiedene Verfahren bekannt, von denen folgende, bezüglich der vorliegenden Erfindung, von Interesse sind.

In der DE-AS 1 295 543 ist ein Verfahren zur Abtrennung von TFE aus den bei der Pyrolyse von Chlordifluormethan oder Bromdifluormethan erhaltenen Gemischen durch extraktive Destillation unter Zusatz von Methanol, Diethylether, Aceton oder Ethylacetat beschrieben. Nach dem Verfahren der DE-PS 1 161 877 werden chlorierte Kohlenwasserstoffe wie Methylenchlorid, Chloroform, Methylchlorid, Tetrachlorkohlenstoff, Dichlorethylen, Trichlorethylen und Tetrachlorethylen verwendet. Diese Verfahren haben den Nachteil, daß die Siedepunkte der Extraktionsmittel sehr viel höher liegen als ihre Anwendungstemperatur bei der Extraktivdestillation. Hierdurch müssen erhebliche Energiemengen zur Wiedergewinnung, Reindarstellung und Kühlung vor Anwendung für die genannten Extraktionsmittel aufgewendet werden, zumal auch die anzuwendenden Mengen groß sind, insbesondere wenn die Extraktivdestillation mit der Hauptmenge des flüssigen Pyrolysekondensates, wie sie im Sumpfprodukt der ersten Kolonne vorliegt, durchgeführt wird. Ebenso werden vergleichsweise größere und damit aufwendigere Vorrichtungen wie Wärmetauscher, Destillationskolonnen und so weiter benötigt.

Aus der EP 38 970-A1 ist ein Verfahren bekannt, wonach das nach der Kondensation der Pyrolysegase anfallende, Stickstoff und Kohlenoxid enthaltende Abgas zur Wiedergewinnung des darin enthaltenen TFE mit Difluormonochlormethan, Tetrafluormonochlorethan oder Hexafluormonochlorpropan gewaschen wird. Die anfallende TFE-haltige Waschflüssigkeit wird in die Verflüssigung der Pyrolyseprodukte zurückgeführt. Von einer Einführung dieser Waschflüssigkeit an bestimmten Stellen während des destillativen Reinigungsprozesses für die flüssigen Pyrolyseprodukte ist nicht die Rede, sie wäre auch unzweckmäßig, da die Waschflüssigkeit neben TFE unerwünschte Pyrolyseprodukte enthält, die stören würden, wenn sie nicht den Reinigungsprozeß von Anfang an durchlaufen.

Es wurde nun ein Verfahren gefunden, das es ermöglicht, aus Pyrolysegasen reines TFE zu gewinnen, wobei ein Destillationshilfsmittel mit niedrigem Siedepunkt verwendet wird, das zu geringerem Energieverbrauch und/oder höherer Reinheit des erzeugten TFE führt und höhere Durchsatzmengen in der destillativen Reinigung ermöglicht. Sofern das zu reinigende Pyrolyseprodukt aus der thermischen Zersetzung von Difluormonochlormethan stammt, ist der Energiebedarf und apparative Aufwand besonders niedrig.

Das neue Verfahren zur Gewinnung von reinem Tetrafluorethylen aus einem Gas, das bei der Pyrolyse von einer Fluorkohlenstoffverbindung mit 1 bis 4 C-Atomen, die ein Wasserstoff-und ein Chloratom enthalten kann, entsteht und das nach Abtrennung von Chlorwasserstoff oder Fluorwasserstoff sowie gegebenenfalls von Wasserdampf 20 bis 70 Gew.-% Tetrafluorethylen enthält,durch Kondensation des

2

Gases gegebenenfalls unter Druck, Destillation des flüssigen Kondensates in mehreren hintereinander geschalteten Kolonnen, wobei aus der in Strömungsrichtung des Pyrolyse-Kondensates ersten Kolonne über Kopf die leichter als Tetrafluorethylen siedenden Verbindungen und azeotropen Gemische und aus der in Strömungsrichtung des Kondensates zweiten Kolonne über Kopf Tetrafluorethylen abgezogen wird, während die schwerer als Tetrafluorethylen siedenden Verbindungen und azeotropen Gemische vom Sumpf der zweiten Kolonne abgezogen und zur Gewinnung weiterer Fluorkohlenstoffverbindungen destillativ aufgearbeitet werden, ist dadurch gekennzeichnet, daß in die zweite Kolonne je 1 kg des aus dieser Kolonne abgezogenen Tetrafluorethylens 0,6 bis 2 kg Difluormonochlormethan oberhalb der Zuführung des Sumpfproduktes der ersten Kolonne eingeführt werden und daß je 1 kg vom Kopf der zweiten Kolonne abgezogenen Tetrafluorethylens 3 bis 10 kg Kopfprodukt der zweiten Kolonne kondensiert und in diese Kolonne zurückgeführt werden.

Für die erfindungsgemäße Herstellung von Tetrafluorethylen durch Pyrolyse sind vorzugsweise folgende Fluorkohlenstoffverbindungen geeignet: Tetrafluormonochlorethan, Trifluormethan und Perfluorcyclobutan. Wegen der günstigen Ergebnisse ist Difluormonochlormethan als Einsatzprodukt für die Pyrolyse besonders bevorzugt. Es können auch Mischungen der genannten Verbindungen als Einsatzprodukt für die Pyrolyse verwendet werden.

Sofern das Einsatzprodukt für die Pyrolyse gebundenen Wasserstoff enthält, entsteht bei der Pyrolyse je nach Art der eingesetzten Verbindung beziehungsweise des Verbindungsgemisches Fluorwasserstoff und/oder Chlorwasserstoff. Diese werden zweckmäßig, wie oben bereits erwähnt, durch Lösen in Wasser oder in einer wäßrigen Lösung, die bereits Fluorwasserstoff beziehungsweise Chlorwasserstoff enthält, entfernt. Das anschließend getrocknete Pyrolysegas soll zweckmäßig 20 bis 70 Gew.-%, bezogen auf das gesamte Gas, TFE enthalten. Prinzipiell sind auch niedrigere Gehalte an TFE möglich, jedoch wird dadurch die Wirtschaftlichkeit des Verfahrens ohne Erreichung von Vorteilen schlechter. Die oben beschriebenen, behandelten Pyrolysegase, die mehr als 70 Gew.-% Tetrafluorethylen enthalten, erfordern in der Pyrolyse in der Regel Bedingungen (zum Beispiel hohe Temperatur), die die Bildung von unerwünschten Nebenprodukten begünstigen, verschlechtern damit die Ausbeute, bezogen auf für die Pyrolyse eingesetzte Fluorkohlenstoffverbindung, und können zu einem ungerechtfertigt hohen Reinigungsaufwand führen.

Die Kondensation des gewaschenen und getrockneten Pyrolysegases erfolgt bei 0,1 bis 1,5 MPa durch Abkühlung auf entsprechende Temperatur. Vorzugsweise wird im Druckbereich von 0,15 bis 0,5 MPa gearbeitet. Letzter Wert sollte auch aus Sicherheitsgründen (Explosionsgefahr) nicht überschritten werden.

Der durch Kondensation verflüssigte Teil des Pyrolysegases wird nun in einer ersten Destillationskolonne, die, um eine ausreichende Trennwirkung zu erzielen, 30 bis 150 theoretische Böden aufweisen sollte, zur Abtrennung der Leichtsieder über Kopf destilliert. Diese Kolonne kann bei einem Druck von 0,3 bis 1,8 MPa betrieben werden, vorzugsweise wird im Druckbereich von 0,4 bis 0,5 MPa gearbeitet, wobei der obere Wert des Vorzugsbereiches, wie oben bereits ausgeführt, durch Sicherheitserwägungen bestimmt ist. Das Kopfprodukt der ersten Kolonne wird, wie auch das nicht kondensierbare Abgas aus der Pyrolyse, nach bekannten Methoden weiterbehandelt, um die darin enthaltenen vergleichsweise geringen Anteile an TFE zu gewinnen. Das Sumpfprodukt der ersten Kolonne wird in eine zweite Kolonne, die ebenfalls zur Erzielung einer ausreichenden Trennwirkung 30 bis 150 theoretische Böden haben sollte, eingeführt und hier über Kopf reines TFE abdestilliert. Die zweite Kolonne kann im Druckbereich von 0,3 bis 1,2 MPa betrieben werden, wobei auch hier ein Bereich von 0,4 bis 0,5 MPa aus den oben erläuterten Gründen bevorzugt ist.

Erfindungsgemäß werden in diese zweite Kolonne oberhalb der Zuführung des Sumpfproduktes aus der ersten Kolonne je 1 kg des aus dieser Kolonne abgezogenen Tetrafluorethylens 0,6 bis 2 kg Difluormonochlormethan als Destillationshilfsmittel eingespeist. Die Höhe der Einspeisung wird zweckmäßig so gewählt, daß ein Übertreten des Difluormonochlormethans zum reinen TFE unter 10 Tpm (= Teile Difluormonochlormethan je 1 Million Teile Rein-TFE) vorzugsweise unter 5 Tpm gehalten wird. Werden unter 0,6 kg Difluormonochlormethan je kg TFE verwendet, wird der erfindungsgemäße Effekt in Kolonnen von industriell ausführbarer Trennstufenzahl nicht mehr erreicht, Zusatzmengen von über 2 kg Difluormonochlormethan je kg TFE sind zwar möglich, bringen aber keine zusätzlichen Vorteile mehr, sondern erhöhen lediglich die Kosten; vorzugsweise werden 0,75 bis 1,5 kg Difluormonochlormethan je kg TFE eingesetzt.

Durch Anwendung des erfindungsgemäßen Verfahrens braucht in der ersten (Leichtsieder) Kolonne nur noch Trifluormethan und Difluorethylen vollständig abgetrennt zu werden, während das mit dem Pyrolysegaskondensat eingetretene Difluormethan sich zwischen Kopf- und Sumpfprodukt verteilen kann. Die geringeren Anforderungen an die Trennung ermöglichen eine Herabsetzung der Rücklaufmenge. Es werden je 1 kg vom Kopf der zweiten Kolonne abgezogenen TFE 6 bis 25 kg, vorzugsweise 6 bis 15 kg, Kopfprodukt der ersten Kolonne kondensiert und in diese Kolonne zurückgeführt. Trotzdem ein Teil des Difluormethans mit dem Sumpfprodukt der ersten Kolonne in die zweite (TFE) Kolonne eingeführt wird, kann auch diese Kolonne mit einem vergleichsweise niedrigeren Rücklaufverhältnis betrieben werden, ohne die Reinheit des erzeugten TFE zu gefährden, da das Difluormethan mit dem Sumpfprodukt aus dieser Kolonne abgeführt wird. Je 1 kg vom Kopf der zweiten Kolonne abgezogenen Tetrafluorethylens werden 3 bis 10 kg, vorzugsweise 3 bis 5 kg Kopfprodukt, der zweiten Kolonne kondensiert und in diese Kolonne zurückgeführt, wenn TFE üblicher Reinheit erzeugt werden soll. Es ist jedoch auch möglich, TFE besonderer Reinheit zu gewinnen, im Bereich der Rückführung von 5 bis 10 kg Kopfprodukt je kg erzeugtes TFE, insbesondere wenn der zweiten Kolonne relativ viel, beispielsweise 1,5 bis 2 kg Difluormonochlormethan je kg erzeugtes TFE zugeführt werden.

Das Sumpfprodukt der zweiten (TFE) Kolonne wird nach bekannten Verfahren weiter destillativ aufgearbeitet, zweckmäßig wird es einer Extraktivdestillation unterworfen, um das azeotrope Gemisch aus Difluormonochlormethan und Hexafluorpropen zu trennen. Hierfür können bekannte Extraktionsmittel wie Methanol, Aceton, Diethylether oder Ethylacetat verwendet werden, wobei wesentlich weniger Extraktionsmittel gebraucht wird und in einem günstigeren Temperaturbereich gearbeitet werden kann, als wenn diese Extraktivdestillation, gemäß dem Stand der Technik, mit dem gesamten Pyrolysegas durchgeführt würde.

Für den Fachmann nicht vorhersehbar ist die Tatsache, daß bei Anwendung des erfindungsgemäßen Verfahrens die Trennwirkung der zweiten (TFE) Kolonne sich verschlechtert, wenn das Rücklaufverhältnis zu hoch (deutlich über 10 kg Kopfprodukt je kg erzeugtes TFE) gewählt wird. Insofern verhält sich die erfindungsgemäße Trennung entgegengesetzt dem, was bei einer normalen destillativen Trennung zu erwarten wäre. Das erfindungsgemäße Verfahren unterscheidet sich von üblichen Absorptions- oder Extraktivdestillationen dadurch, daß das Destillationshilfsmittel in vergleichsweise nur geringen Mengenverhältnissen vorliegt.

Wie bereits oben erwähnt, ermöglicht es das erfindungsgemäße Verfahren, Tetrafluorethylen in guter Ausbeute und Reinheit mit verminderten Energie- und apparativen Kosten herzustellen. Mit besonderem Vorteil wird das neue Verfahren eingesetzt zur Gewinnung von TFE aus Pyrolysegasen, die durch thermische Zersetzung von Difluormonochlormethan oder Stoffgemischen, die diesen Stoff überwiegend enthalten, erzeugt wurden.

Nachfolgende Beispiele sollen die Erfindung näher erläutern:

Beispiel 1

Gasförmiges Difluor-monochlormethan wird in einem elektrisch beheizten Rohr thermisch gespalten. Die Spaltgase verlassen das Rohr mit einer Temperatur von 730 °C, sie werden anschließend mit einer wäßrigen Lösung, die 30 Gew.-%, bezogen auf die Lösung, Chlorwasserstoff enthält und die mit Kühlwasser von 12 °C gekühlt wird, gewaschen, dann durch gekühltes Wasser, danach durch auf 12 °C gekühlte, verdünnte Natronlauge geleitet und schließlich mit konzentrierter Schwefelsäure getrocknet. Nach der Trocknung werden die Spaltgase bei -80 °C kondensiert, das Abgas verworfen.

Es werden 2 705 g/h Kondensat erhalten, das nach gaschromatographischer Analyse folgende Zusammensetzung aufweist:

```
Difluorethylen                      0,47 g/h
Trifluormethan                      4,19 g/h
Tetrafluorethylen               1 025,21 g/h
Difluormethan                       0,30 g/h
Trifluorethylen                     0,83 g/h
Difluormonochlormethan          1 095,81 g/h
Rest,bestehend im
wesentlichen aus
Hexafluorpropylen           )
Tetrafluormonochlorethan    )
                                  578,16 g/h
Perfluorcyclobutan und      )
Hexafluormonochlorpropan    )
```

Dieses Kondensat wird in einer ersten Kolonne von 25 mm innerem Durchmesser, die mit Metallringen gefüllt ist und eine theoretische Bodenzahl von 84 aufweist, unter einem Druck von 0,5 MPa destilliert, wobei 6 700 g/h des kondensierten Kopfproduktes in die Kolonne zurückgeführt und 81,4 g/h des Kopfproduktes abgeführt werden. Das abgeführte Kopfprodukt hat nach gaschromatographischer Analyse folgende Zusammensetzung:

Difluorethylen          0,47 g/h
Trifluormethan          4,19 g/h
Tetrafluorethylen          76,66 g/h
Difluormethan          0,07 g/h

Es kann zur Gewinnung des Tetrafluorethylens nach bekannten Reinigungs-Verfahren aufgearbeitet werden.

Das Sumpfprodukt der ersten Kolonne wird in einer zweiten Kolonne von 25 mm innerem Durchmesser, die mit Metallringen gefüllt ist und eine theoretische Bodenzahl von 84 aufweist, erneut unter einem Druck von 0,5 MPa destilliert. In der Hälfte der Gesamtlänge dieser Kolonne werden 600 g/h Difluormonochlormethan eingeführt. Der Rücklauf wird so eingestellt, daß das Kopfprodukt in 100 g Tetrafluorethylen nicht mehr als je 0,5 mg Difluormethan beziehungsweise Trifluorethylen enthält. Dies wird erreicht, wenn 3 300 g/h des kondensierten Kopfproduktes in die zweite Kolonne zurückgeführt werden, 948,4 g/h des Kopfproduktes werden abgeführt. Es enthält nach gaschromatographischer Analyse

Tetrafluorethylen          948,4 g/h
Difluormethan          0,0047 g/h (< 0,0005 g/100 g TFE)
Trifluorethylen          < 0,0005 g/h

2 275,2 g Sumpfprodukt werden aus der zweiten Kolonne abgeführt, es hat nach gaschromatographischer Analyse folgende Zusammensetzung

```
Tetrafluorethylen                          0,15 g/h

Difluormethan                              0,23 g/h

Trifluorethylen                            0,83 g/h

Difluormonochlormethan                 1 695,81 g/h

Rest,bestehend im

wesentlichen aus

Hexafluorpropylen              )

Tetrafluormonochlorethan       )
                                       578,16 g/h
Perfluorcyclobutan und         )

Hexafluormonochlorpropan       )
```

Das Sumpfprodukt kann zur Gewinnung des Difluormonochlormethans, Hexfluorpropylens und anderer Produkte weiteren Destillationsschritten unterworfen werden. Hierbei kann das Difluormonochlormethan in einem Temperaturbereich durch Destillation gewonnen werden, der die Verwendung von Wasserdampf als Heizmittel und Wasser als Kühlmittel erlaubt, wobei nur etwa 1/5 der Energiekosten aufgewendet werden müssen, als bei der Tieftemperatur-Destillation der Tetrafluorethylen enthaltenden Stoffgemische. Das nach Abtrennung von Chlorwasserstoff, Fluorwasserstoff und Wasserdampf erhaltene Spaltgas-Kondensat enthält 38,0 Gew.-% Tetrafluorethylen. Es werden je 1 kg des aus der zweiten Kolonne abgezogenen Tetrafluorethylens

0,63 kg          Difluormonochlormethan in die zweite Kolonne eingeführt,
7 kg          kondensiertes Kopfprodukt der ersten Kolonne in diese zurückgeführt und
3,5 kg          kondensiertes Kopfprodukt der zweiten Kolonne in diese zurückgeführt.

Vergleichsversuch

Es wird gearbeitet wie in Beispiel 1 beschrieben, jedoch wird in die zweite Kolonne kein Difluormonochlormethan eingespeist. Das Rücklaufverhältnis der ersten und zweiten Kolonne wird wiederum so eingestellt, daß das Kopfprodukt der zweiten Kolonne je 100 g Tetrafluorethylen nicht mehr als je 0,5 mg

EP 0 307 673 B1

Difluormethan beziehungsweise Trifluorethylen enthält. Hierzu müssen 19 100 g/h kondensiertes Kopfprodukt der ersten Kolonne in diese Kolonne und 7 600 g/h kondensiertes Kopfprodukt der zweiten Kolonne in diese Kolonne zurückgeführt werden. Am Kopf der ersten Kolonne werden 80,2 g/h ausgetragen.

Zusammensetzung:

| | |
|---|---|
| Difluorethylen | 0,57 g/h |
| Trifluormethan | 4,17 g/h |
| Tetrafluorethylen | 75,16 g/h |
| Difluormethan | 0,29 g/h |

Am Kopf der zweiten Kolonne werden 953,7 g/h ausgetragen.

Zusammensetzung:

| | |
|---|---|
| Tetrafluorethylen | 953,7 g/h |
| Difluormethan | 0,0048 g/h (0,0005 g/100 g TFE) |
| Trifluorethylen | < 0,0005 g/h |

Aus dem Sumpf der zweiten Kolonne werden 1 676,1 g/h ausgetragen.

Zusammensetzung:

```
Tetrafluorethylen              0,35  g/h

Difluormethan                  0,01  g/h

Trifluorethylen                0,85  g/h

Difluormonochlormethan     1 095,81  g/h


Rest,bestehend im

wesentlichen aus

Hexafluorpropylen            )

Tetrafluormonochlorethan )

Perfluorcyclobutan und       )

Hexafluormonochlorpropan )
```
                                579,1  g/h

Das Spaltgaskondensat enthält 38,0 Gew.-% Tetrafluorethylen. Es werden je 1 kg des aus der zweiten Kolonne abgezogenen Tetrafluorethylens

20 kg kondensiertes Kopfprodukt der ersten Kolonne in diese zurückgeführt
8 kg kondensiertes Kopfprodukt der zweiten Kolonne in diese zurückgeführt.

Der Energieaufwand um bei der Tieftemperatur-Destillation in der ersten und zweiten Kolonne im Vergleichsversuch wesentlich höhere Rücklauf-Verhältnisse,als bei dem erfindungsgemäßen Beispiel fahren zu müssen, ist erheblich, demgegenüber fällt die Energieeinsparung im Vergleichsversuch,bei dem 600 g/h Difluormonochlormethan weniger zurückgewonnen werden müssen, kaum ins Gewicht, da diese Destillation in einem energetisch wesentlich günstigeren Temperaturbereich vorgenommen werden kann.

Beispiel 2

Es wird gearbeitet wie in Beispiel 1 beschrieben, mit folgenden Unterschieden:
Die zweite Kolonne hat einen inneren Durchmesser von 25 mm und ist mit Metallringen gefüllt, ist aber kürzer und weist nur eine theoretische Bodenzahl von 63 auf. In der Hälfte der Gesamtlänge dieser Kolonne werden 1 140 g/h Difluormonochlormethan eingeführt. Wiederum wird das Rücklaufverhältnis der ersten und

6

zweiten Kolonne so eingestellt, daß das Kopfprodukt der zweiten Kolonne je 100 g Tetrafluorethylen nicht mehr als je 0,5 mg Difluormethan beziehungsweise Trifluorethylen enthält. Hierzu müssen 6 600 g/h kondensiertes Kopfprodukt der ersten Kolonne in diese Kolonne und 3 800 g/h kondensiertes Kopfprodukt der zweiten Kolonne in diese Kolonne zurückgeführt werden. Am Kopf der ersten Kolonne werden 82,5 g/h ausgetragen.

Zusammensetzung:

| | |
|---|---|
| Difluorethylen | 0,47 g/h |
| Trifluormethan | 4,19 g/h |
| Tetrafluorethylen | 77,76 g/h |
| Difluormethan | 0,07 g/h |

Am Kopf der zweiten Kolonne werden 951,3 g/h ausgetragen.

Zusammensetzung:

| | |
|---|---|
| Tetrafluorethylen | 951,3 g/h |
| Difluormethan | 0,0046 g/h (< 0,0005 g/100 g TFE) |
| Trifluorethylen | < 0,0005 g/h |

Aus dem Sumpf der zweiten Kolonne werden 2 812,3 g/h ausgetragen.

Zusammensetzung:

| | |
|---|---|
| Tetrafluorethylen | 0,15 g/h |
| Difluormethan | 0,24 g/h |
| Trifluorethylen | 0,81 g/h |
| Difluormonochlormethan | 2 233,60 g/h |
| Rest, bestehend im wesentlichen aus Hexafluorpropylen ) Tetrafluormonochlorethan ) Perfluorcyclobutan ) Hexafluormonochlorpropan ) | 577,50 g/h |

Das Spaltgaskondensat enthält 38,0 Gew.-% Tetrafluorethylen. Es werden je 1 kg des aus der zweiten Kolonne abgezogenen Tetrafluorethylens

| | |
|---|---|
| 1,2 kg | Difluormonochlormethan in die zweite Kolonne eingeführt |
| 6,9 kg | kondensiertes Kopfprodukt der ersten Kolonne in diese zurückgeführt und |
| 4 kg | kondensiertes Kopfprodukt der zweiten Kolonne in diese zurückgeführt. |

**Patentansprüche**

1. Verfahren zur Gewinnung von reinem Tetrafluorethylen aus einem Gas, das bei der Pyrolyse von einer Fluorkohlenstoffverbindung mit 1 bis 4 C-Atomen, die ein Wasserstoff- und ein Chloratom enthalten kann, entsteht und das nach Abtrennung von Chlorwasserstoff oder Fluorwasserstoff sowie gegebenenfalls von Wasserdampf 20 bis 70 Gew.-% Tetrafluorethylen enthält, durch Kondensation des Gases gegebenenfalls unter Druck, Destillation des flüssigen Kondensates in mehreren hintereinandergeschalteten Kolonnen, wobei aus der in Strömungsrichtung des Pyrolysekondensates ersten Kolonne über Kopf die leichter als Tetrafluorethylen siedenden Verbindungen und azeotropen Gemische und aus der in Strömungsrichtung des Kondensates zweiten Kolonne über Kopf Tetrafluorethylen abgezogen wird, während die schwerer als Tetrafluorethylen siedenden Verbindungen und azeotropen Gemische vom Sumpf der zweiten Kolonne abgezogen und zur Gewinnung weiterer Fluorkohlenstoffverbindungen destillativ aufgearbeitet werden, dadurch gekennzeichnet, daß in die zweite Kolonne je 1 kg des aus dieser Kolonne abgezogenen Tetrafluorethylens 0,6 bis 2 kg Difluormonochlormethan oberhalb der Zuführung des Sumpfproduktes der ersten Kolonne eingeführt werden und daß je 1 kg vom Kopf der zweiten Kolonne abgezogenen Tetrafluorethylens 3 bis 10 kg Kopfprodukt der zweiten Kolonne kondensiert und in diese Kolonne zurückgeführt werden.

2. Verfahren nach Anspruch 1, dadurch gekennzeichnet, daß je 1 kg vom Kopf der zweiten Kolonne abgezogenen Tetrafluorethylens 3 bis 5 kg Kopfprodukt der zweiten Kolonne kondensiert und in diese Kolonne zurückgeführt werden.

3. Verfahren nach Anspruch 1 oder 2, dadurch gekennzeichnet, daß je 1 kg vom Kopf der zweiten Kolonne abgezogenen Tetrafluorethylens 6 bis 15 kg Kopfprodukt der ersten Kolonne kondensiert und in diese Kolonne zurückgeführt werden.

4. Verfahren nach einem oder mehreren der Ansprüche 1 bis 3, dadurch gekennzeichnet, daß die erste und zweite Kolonne im Druckbereich von 0,4 bis 0,5 MPa betrieben werden.

5. Verfahren nach einem oder mehreren der Ansprüche 1 bis 4, dadurch gekennzeichnet, daß ein Kondensat aus einem Gas eingesetzt wird, das durch Pyrolyse von Difluormonochlormethan erzeugt wurde.

**Claims**

1. A process for the production of pure tetrafluoroethylene from gas formed in the pyrolysis of a fluorocarbon compound having 1 to 4 carbon atoms, which can contain a hydrogen and a chlorine atom, which gas contains 20 to 70 % by weight of tetrafluoroethylene after the removal of hydrogen chloride or hydrogen fluoride and possibly of steam, by condensation of the gas, if necessary, under pressure, distillation of the liquid condensate in several columns connected in series, the compounds and azeotropic mixtures boiling at a lower temperature than tetrafluoroethylene being discharged at the top from the first column in the flow direction of the pyrolysis condensate and tetrafluoroethylene being discharged at the top from the second column in the flow direction of the condensate while the compounds and azeotropic mixtures boiling at a higher temperature than tetrafluoroethylene are discharged from the bottom of the second column and are worked up by distillation to produce further fluorocarbon compounds, characterized by introducing into the second column above the inlet for the bottom product of the first column 0.6 to 2 kg of difluoromonochloromethane per 1 kg of tetrafluoroethylene discharged from the second column and by condensing 3 to 10 kg per 1 kg tetrafluoroethylene of top product of the second column and recycling it into this column.

2. The process as claimed in claim 1, characterized in that 3 to 5 kg of top product of the second column per 1 kg of tetrafluoroethylene discharged at the top of the second column are condensed and recycled into this column.

3. The process as claimed in claim 1 or 2, characterized in that 6 to 15 kg of top product of the first column per 1 kg of tetrafluoroethylene discharged from the top of the second column are condensed and recycled into the first column.

4. The process as claimed in one or more of claims 1 to 3, characterized in that the first and second column are operated in the pressure range from 0.4 to 0.5 MPa.

5. The process as claimed in one or more of claims 1 to 4, characterized in that a condensate from a gas is used which had been generated by pyrolysis of difluoromonochloromethane.

**Revendications**

1. Procédé pour obtenir des tétrafluoréthylènes purs à partir d'un gaz qui se forme lors de la pyrolyse d'un composé fluorocarboné renfermant de 1 à 4 atomes de carbone et, éventuellement, un atome d'hydrogène et un atome de chlore, et qui contient, après séparation du chlorure d'hydrogène ou du fluorure d'hydrogène ainsi qu'éventuellement de la vapeur d'eau, de 20 à 70 % en poids de tétrafluoréthylène, par condensation du gaz éventuellement sous pression, distillation du condensat liquide dans plusieurs colonnes montées en série, les composés et mélanges azéotropes plus volatils que le tétrafluoréthylène étant retirés à la tête de la première colonne (dans le sens de l'écoulement du condensat de pyrolyse) et le tétrafluoréthylène étant retiré à la tête de la deuxième colonne (dans le sens de l'écoulement du condensat) tandis que les composés et mélanges azéotropes moins volatils que le tétrafluoréthylène sont retirés du bas de la deuxième colonne et soumis à un traitement complémentaire par distillation en vue de l'obtention d'autres composés fluorocarbonés, procédé caractérisé en ce qu'on introduit dans la deuxième colonne, par kilogramme du tétrafluoréthylène retiré de cette colonne, de 0,6 à 2 kg de difluoromonochlorométhane au-dessus de l'endroit d'arrivée du produit venant du bas de la première colonne et en ce qu'on condense, par kilogramme du tétrafluoréthylène retiré à la tête de la deuxième colonne, de 3 à 10 kg du produit de tête de la deuxième colonne et on les renvoie dans cette colonne.

2. Procédé selon la revendication 1, caractérisé en ce qu'on condense, par kilogramme du tétrafluoréthylène retiré à la tête de la deuxième colonne, de 3 à 5 kg du produit de tête de la deuxième colonne et on les renvoie dans cette colonne.

3. Procédé selon la revendication 1 ou 2, caractérisé en ce qu'on condense, par kilogramme du tétrafluoréthylène retiré à la tête de la deuxième colonne, de 6 à 15 kg du produit de tête de la première colonne et on les renvoie dans cette colonne.

4. Procédé selon une ou plusieurs des revendications 1 à 3, caractérisé en ce qu'on fait fonctionner la première et la deuxième colonne dans un intervalle de pression allant de 0,4 à 0,5 MPa.

5. Procédé selon une ou plusieurs des revendications 1 à 4, caractérisé en ce qu'on utilise un condensat provenant d'un gaz qui a été produit par pyrolyse du difluoromonochlorométhane.